# NEUE EUROPÄISCHE PATENTSCHRIFT

(11) **EP 2 593 707 B2**
(45) Veröffentlichungstag und Bekanntmachung des Hinweises auf die Entscheidung über den Einspruch: **05.05.2021**
(45) Hinweis auf die Patenterteilung: 17.10.2018
(21) Anmeldenummer: 11732387.3
(22) Anmeldetag: 14.07.2011
(51) Int. Cl.: F16M 11/04

(54) **HALTEVORRICHTUNG FÜR EIN INSTRUMENT**
RETAINING DEVICE FOR AN INSTRUMENT
DISPOSITIF DE RETENUE POUR UN INSTRUMENT

(30) Priorität: 15.07.2010 DE 102010027248
(43) Veröffentlichungstag der Anmeldung: 22.05.2013
(73) Patentinhaber: SENSODRIVE GmbH, 82234 Weßling (DE)
(72) Erfinder: SPORER, Norbert, 82549 Koenigsdorf (DE); HÄHNLE, Matthias, 80634 München (DE)
(74) Vertreter: Beyer, Andreas
(86) Internationale Anmeldenummer: PCT/EP2011/003519
(87) Internationale Veröffentlichungsnummer: WO 2012/007168

(56) Entgegenhaltungen:
- EP-A1- 2 113 344
- EP-A2- 1 520 548
- DE-A1-102004 008 381
- DE-A1-102004 008 381
- DE-A1-102008 027 008
- DE-T5- 10 296 748
- JP-A- 2001 025 472
- US-A1- 2007 156 122
- US-A1- 2007 156 122
- FRANCOIS PIERROT et al.: "Hippocrate: a Safe Robot Arm for Medical Applications with Force Feedback", Medical Image Analysis, vol. 3, no. 3, September 1999 (1999-09), pages 285-300,
- MaschinenÃ¼bersetzung von JPA 2001025472
- Human Übersetzung des Zusammenfassung und der Absätze (0010) und (0042) von JPA 2001025472.

## Beschreibung

Die vorliegende Erfindung betrifft eine Haltevorrichtung für ein Instrument, die wenigstens ein Gelenk mit wenigstens zwei relativ zueinander beweglichen Teilen und wenigstens eine dem Gelenk zugeordnete Antriebseinheit aufweist. Die Antriebseinheit ist dazu ausgebildet, das Gelenk zu bewegen, indem sie wenigstens eines der zueinander beweglichen Teile des Gelenks antreibt.

Derartige angetriebene Haltevorrichtungen sind aus dem Stand der Technik bereits bekannt. Je nach Art der Anwendung kann eine solche Haltevorrichtung eine sogenannte Stativvorrichtung sein, die ein medizinisch-optisches Instrument hält, oder ein Roboter mit einer Manipulationseinheit als Instrument. Das Gelenk ist in der Regel als Drehgelenk ausgebildet, wobei durch die zueinander beweglichen Teile des Gelenks und durch das Instrument ein Last-Drehmoment in dem Drehgelenk erzeugt wird. Die Antriebseinheit kann ein entsprechendes Gegendrehmoment in das Drehgelenk einleiten, welches dazu geeignet ist, das Last-Drehmoment zu kompensieren.

Eine derartige Haltevorrichtung ist beispielsweise aus dem Dokument DE 10 2004 063 606 A1 bekannt, wobei die Haltevorrichtung einen Elektromotor zum Ausgleich des Last-Drehmoments umfasst, der mit Mitteln zum Erfassen der Stellung des Drehgelenks kombiniert ist und in Abhängigkeit einer erfassten Drehgelenkstellung zur Erzeugung eines das Last-Drehmoment ausgleichenden Gegenmoments bestromt werden kann. Hierzu ist eine Steuereinheit vorgesehen, die den erforderlichen Motorstrom einstellt.

Aus der EP 1 520 548 A2 ist eine Haltevorrichtung für medizinische Geräte bekannt, die mehrere Gelenke aufweist. Jedes Gelenk kann mittels einer federbelasteten Bremseinrichtung wahlweise arretiert werden. Die Vorspannung der Feder und damit die Arretierkraft ist über eine von einem Elektromotor angetriebene Verstelleinrichtung einstellbar.

Sogenannte Stativvorrichtungen oder Haltevorrichtungen zum Halten eines medizinisch-optischen Instruments werden üblicherweise eingesetzt, um dem Operateur oder weiterem medizinischem Personal eine möglichst einfache Handhabung des Instruments zu ermöglichen. So kann mittels der Haltevorrichtung das daran angebrachte medizinisch-optische Instrument von Hand in eine gewünschte Position gebracht werden, ohne während des Positioniervorgangs das Eigengewicht des Instruments halten zu müssen, und die Haltevorrichtung hält das Instrument nach Abschluss des Positioniervorgangs in der gewünschten Stellung.

Üblicherweise weist die Haltevorrichtung eine Sicherungseinrichtung auf, die sicherstellt, dass das Instrument und die Haltevorrichtung während des Positioniervorgangs verhältnismäßig leicht bewegbar sind, und die nach Abschluss des Positioniervorgangs eine Bewegung der Haltevorrichtung blockiert. Hierzu kann, wie im Dokument DE 10 2004 063 606 A1 angegeben, eine Magnetbremse in dem Drehgelenk vorgesehen sein, die je nach Ansteuerung eine Bewegung der zueinander beweglichen Teile des Drehgelenks freigibt oder unterbindet. Wenn ein Bediener die Haltevorrichtung zusammen mit dem Instrument bewegen möchte, löst er zuerst die Magnetbremse, indem er beispielsweise auf einen Freigabeknopf drückt, und bewegt dann das Instrument von Hand in die gewünschte Position. Sobald er den Freigabeknopf loslässt, fällt die Magnetbremse automatisch wieder ein und blockiert eine weitere Bewegung der zueinander beweglichen Teile des Drehgelenks, so dass die Haltevorrichtung nun das Instrument in der zu diesem Zeitpunkt erreichten Stellung hält.

Während der Bediener den Positioniervorgang durchführt und den Knopf zur Freigabe der Magnetbremse gedrückt hält, wirkt der Elektromotor bzw. allgemein eine Antriebseinheit in der Weise auf die beweglichen Teile des Drehgelenks ein, dass ein in das Drehgelenk eingeleitetes Last-Drehmoment durch ein Gegendrehmoment ausgeglichen wird, so dass das Instrument für den Bediener quasi schwerelos verfahrbar ist.

Durch eine solche Sicherungseinrichtung wird ermöglicht, dass ein menschlicher Bediener, wie beispielsweise ein Operateur, und eine angetriebene Haltevorrichtung gemeinsam eingesetzt werden können, ohne dass die Haltevorrichtung hierbei ein Sicherheitsrisiko für die in dem Arbeitsbereich der Haltevorrichtung befindlichen Menschen darstellt. Das automatische Einfallen der Bremsen, sobald der Bediener die Sicherungseinrichtung nicht mehr betätigt, verhindert, dass die Haltevorrichtung sich unerwünscht in eine Position bewegen kann, in der sie einen Menschen verletzen kann. Die Bremswirkung der Bremsen ist hierbei so gewählt, dass die Antriebskraft der Antriebseinheit nicht ausreicht, um die Teile eines Gelenks, an dem die Bremse eingefallen ist, relativ zu einander zu bewegen.

Ein Problem, das im Zusammenhang mit Haltevorrichtungen wie auch allgemein mit Robotereinheiten aller Art bekannt geworden ist, besteht in der Schwingungsdämpfung der Anordnung. Grundsätzlich können sowohl unerwünschte Nachschwingungen nach Abschluss des Positioniervorgangs der Haltevorrichtung in diese und das damit verbundene Instrument eingeleitet werden, als auch äußere Gebäudeschwingungen, die je nach Art der Aufstellung der Haltevorrichtung über die Raumdecke, Seitenwände des Raums oder den Raumboden auf die Haltevorrichtung übertragen werden können. Weiterhin werden auch durch einen Bediener, der an die Haltevorrichtung stößt, beispielsweise wenn er das an der Haltevorrichtung angebrachte Instrument verwenden möchte (z.B. durch ein Mikroskop blicken möchte), unerwünschte Schwingungen in die Haltevorrichtung und das Instrument eingeleitet. Um derartige unerwünschte und störende Schwingungen des Instruments an der Haltevorrichtung möglichst zu eliminieren, ist es aus dem Stand der Technik bekannt, zusätzliche schwingungsdämpfende Maßnahmen zu ergreifen.

Eine erste bekannte Maßnahme besteht darin, die Haltevorrichtung von dem Raum, in dem sie angeordnet ist, schwingungsdämpfend zu entkoppeln, beispielsweise durch dämpfende Zwischenelemente, die zwischen der Wand, an der die Haltevorrichtung angebracht ist, und der Haltevorrichtung selbst angeordnet sein können. Derartige Zwischenelemente können sehr aufwändig und kostspielig sein, insbesondere wenn sie dazu geeignet sein sollen, Schwingungen unterschiedlichster Frequenz zu dämpfen. Zudem lassen sich auf diese Weise nur Gebäudeschwingungen dämpfen, nicht jedoch die vorstehend angesprochenen Nachschwingungen der Haltevorrichtung.

Als weitere Maßnahme ist es aus dem Stand der Technik bekannt, Systeme zur aktiven Schwingungsdämpfung in Kombination mit der Halteeinrichtung einzusetzen. So offenbart die veröffentlichte deutsche Patentanmeldung DE 10 2004 063 606 A1 eine Einrichtung zur aktiven Schwingungsdämpfung, die einen Sensor zum Erfassen von Schwingungen der Haltevorrichtung aufweist, der eine Regelgröße für einen Schwingungsdämpfungs-Regelkreis bereitstellt. Dieser Schwingungsdämpfungs-Regelkreis gibt als Stellgröße einen Überlagerungs-Motorstrom auf den Elektromotor aus, um damit das Drehgelenk so zu bewegen, dass einer erfassten Schwingung oder Vibration der Haltevorrichtung entgegen gewirkt wird. Nachteilig bei einer solchen Gestaltung ist jedoch, dass bei dieser Art der Schwingungsdämpfung nur so lange Schwingungen bzw. Vibrationen kompensiert werden können, wie die Bremse zum Sichern der Position der Haltevorrichtung das Drehgelenk nicht blockiert.

Jedoch kann es gerade dann erwünscht oder notwendig sein, Schwingungen der Haltevorrichtung und somit auch des Instruments zu dämpfen, wenn sich die Haltevorrichtung in ihrer Haltestellung befindet (d.h. wenn die Bremsen das wenigstens eine Drehgelenk der Haltevorrichtung blockieren).

Daher ist es aus dem Stand der Technik weiterhin bekannt, die Schwingungsdämpfung über zusätzliche Aktuatoren oder Antriebsvorrichtungen zu ermöglichen, die von der Antriebseinheit zum Bewegen des Drehgelenks verschieden sind. So ist beispielsweise aus dem Dokument DE 10 2004 004 602 A1 bekannt, eine Stativvorrichtung für ein medizinisch-optisches Instrument mit einem zusätzlichen Antrieb zu versehen, um eine Schwingungsdämpfung zu ermöglichen, wobei der Antrieb beispielsweise mit einem Steuer- oder Regelkreis verbunden sein kann.

Das Dokument DE 10 2007 034 286 A1 offenbart eine aktive Schwingungsdämpfungsvorrichtung für ein Stativ, welche wenigstens einen Schwingungsaufnehmer zum Aufnehmen einer zu dämpfenden Schwingung und wenigstens einen zusätzlichen Aktuator zum Erzeugen einer dämpfenden Gegenschwingung umfasst, wobei der separate Aktuator beispielsweise Piezo-Elemente umfassen kann.

Eine ähnliche Anordnung ist ebenfalls aus dem Dokument EP 1 447 700 A2 bekannt, bei der ein ARES- (*actively reacting flexible structure*) Bauelement als selbstregulierendes Bauelement eingesetzt wird, das aufgrund der Messung von Schwingungen integrierte Antriebselemente, beispielsweise Piezo-Aktuatoren, so ansteuert, dass diese der Schwingung in Istzeit entgegenwirken, so dass die Schwingungen nicht zu einer Lageveränderung der Haltevorrichtung führen.

Das Vorsehen derartiger, zusätzlicher Antriebselemente oder Dämpfungselemente bedeutet jedoch in jedem Fall die Notwendigkeit, zusätzliche Elemente in die Ausgestaltung der Haltevorrichtung konstruktiv integrieren zu müssen. Diese ziehen nicht nur im Rahmen des Konstruktionsprozesses, sondern auch während der Herstellung und Montage einen erhöhten Aufwand und damit erhöhte Kosten nach sich. Weiterhin kann damit ein erhöhter Inspektions- und/oder Reparaturaufwand verbunden sein, da diese zusätzlichen Elemente regelmäßig auf ihre Funktionsfähigkeit hin kontrolliert werden müssen bzw. im Falle eines Defekts ausgetauscht oder repariert werden müssen.

Somit besteht eine Aufgabe der vorliegenden Erfindung darin, eine kostengünstige und zuverlässige Schwingungsdämpfung der Haltevorrichtung zu ermöglichen, ohne dass auf eine durch die Sicherungseinrichtung bereitgestellte Absicherung der im Arbeitsraum der Haltevorrichtung befindlichen Menschen verzichtet werden muss.

Diese Aufgabe wird durch eine Haltevorrichtung für ein Instrument mit den im Patentanspruch 1 genannten Merkmalen gelöst.

Die Sicherungseinrichtung der erfindungsgemäßen Haltevorrichtung bietet somit die gewünschte Sicherheit, die allgemein bei einem Zusammenspiel von Mensch und Roboter erforderlich ist, indem sich der Roboter bzw. die Haltevorrichtung nur dann völlig frei bewegen lässt, wenn die Freigabeeinheit aktiviert ist, d.h. beispielsweise indem ein Anwender oder Bediener einen Freigabeknopf gedrückt hält. Wird die Freigabeeinheit wieder deaktiviert, so ist die durch die Antriebseinheit bewirkte, d.h. die angetriebene, Bewegung des Gelenks aufgrund der speziell ausgebildeten Sicherungseinrichtung nur noch innerhalb eines vorgegebenen Bewegungsbereichs aus der jeweiligen Arretierposition heraus möglich, d.h. aus der Position heraus, in der sich das Gelenk befunden hat, als die Freigabeeinheit deaktiviert wurde.

Auf diese Weise kann beispielsweise eine Schwingungsdämpfung mit Hilfe der dem Gelenk zugeordneten Antriebseinheit auch dann noch erfolgen, wenn die Haltevorrichtung arretiert ist, da hierfür eine minimale Bewegung des Gelenks innerhalb des vorgegebenen Bewegungsbereichs ausreicht. Gleichzeitig stellt der vorgegebene Bewegungsbereich jedoch auch sicher, dass die Haltevorrichtung im arretierten Zustand keine unkontrollierte Bewegung durchführen kann, die die Sicherheit der im Arbeitsraum der Haltevorrichtung befindlichen Menschen gefährden könnte.

Der Begriff "Instrument" umfasst hierbei sowohl optische und/oder medizinische Instrumente und dergleichen als auch eine Manipulationseinheit eines Roboters. Weiterhin kann der Einsatz einer erfindungsgemäßen Haltevorrichtung selbstverständlich auch dann sinnvoll sein, wenn an der Haltevorrichtung kein Instrument angebracht ist.

Die Arretierung umfasst ferner jede kraft- und/oder formschlüssige Art der Verbindung, die die zueinander beweglichen Teile des Gelenks miteinander oder die Antriebseinheit mit einem Teil des Gelenks verbinden kann, der nicht von der Antriebseinheit angetrieben wird. Weitere Ausgestaltungen, bei denen die Arretierung beispielsweise die Antriebseinheit kraft- oder formschlüssig mit einem anderen unbeweglichen Teil der Haltevorrichtung verbindet, um diese zu blockieren, sind ebenfalls denkbar.

Das Gelenk kann als Drehgelenk ausgebildet sein, bei dem sich wenigstens eines der zueinander beweglichen Teile um eine Drehachse des Drehgelenks verschwenken lässt. Alternativ kann das Gelenk jedoch auch als Schubgelenk ausgebildet sein, das eine lineare Bewegung wenigstens eines der zueinander beweglichen Teile ermöglicht. Grundsätzlich ist auch die Ausbildung als ein Gelenk denkbar, das sowohl eine lineare Bewegung als auch eine Rotationsbewegung eines der zueinander beweglichen Teile des Gelenks zulässt, beispielsweise als Schraubgelenk. In der Praxis werden bislang jedoch bevorzugt Dreh- oder Schubgelenke verwendet und gegebenenfalls miteinander kombiniert.

Die Freigabeeinheit kann einen Handgriff, einen Druckknopf oder jede andere Art der Eingabeeinheit umfassen, die die Absicht eines Benutzers, die Haltevorrichtung zu bewegen, erkennen lässt.

Die Antriebseinheit der vorliegenden Erfindung dient zum Bewegen des Gelenks, indem sie zumindest eines der zueinander beweglichen Teile des Gelenks antreibt. Daher kann die Antriebseinheit - wie aus dem Stand der Technik bekannt - dazu genutzt werden, ein an dem als Drehgelenk ausgebildeten Gelenk angreifendes Last-Drehmoment durch Einleiten eines Gegendrehmoments zu kompensieren. Alternativ, d.h. bei einer mechanischen Voll- oder Teilkompensation eines derartigen Last-Drehmoments, bei der die Kompensation durch die konstruktive Ausgestaltung der Haltevorrichtung erfolgt, oder zusätzlich kann die Antriebseinheit jedoch auch zur Schwingungsdämpfung genutzt werden.

Demgemäß kann die Haltevorrichtung ferner eine Einrichtung zur aktiven Schwingungsdämpfung aufweisen, die dazu ausgebildet ist, ein Ausgleichsignal an die Antriebseinheit auszugeben, welches bewirkt, dass die Antriebseinheit das Gelenk derart bewegt, dass Schwingungen der Haltevorrichtung ausgeglichen werden können. Hierbei können sowohl Drehschwingungen in einem als Drehgelenk ausgebildeten Gelenk als auch Schwingungen in einem als Schubgelenk ausgebildeten Gelenk ausgeglichen werden.

Die Antriebseinheit kann in bekannter Weise von einer Steuereinheit gesteuert werden, die zum Ausgleich eines Last-Drehmoments, das in dem als Drehgelenk ausgebildeten Gelenk durch das Instrument selbst und/oder die beweglichen Teile des Drehgelenks hervorgerufen wird, ein Steuersignal an die Antriebseinheit ausgibt. Dieses bewirkt, dass die Antriebseinheit in dem Drehgelenk ein das Last-Drehmoment ausgleichendes Gegenmoment erzeugt (aktive Kompensation).

Ebenso kann bei einer Ausbildung des Gelenks als Schubgelenk die Antriebseinheit von der Steuereinheit gesteuert eine Gegenkraft zum Ausgleich einer durch die Eigengewichtskraft der zueinander beweglichen Gelenkteile und damit verbundener Elemente der Haltevorrichtung hervorgerufenen Zug- oder Druckkraft in dem Gelenk erzeugen.

Wird die Antriebseinheit bereits aktiv zur Erzeugung eines solchen Gegenmoments oder einer solchen Gegenkraft genutzt, so gibt die Einrichtung zur aktiven Schwingungsdämpfung ein Ausgleichsignal an die Antriebseinheit aus, welches das Steuersignal überlagert und auf diese Weise die Schwingungsdämpfung bewirkt.

Derselbe schwingungsdämpfende Effekt kann jedoch auch bei einer Haltevorrichtung erzielt werden, die keine aktive Kompensation mit Hilfe der Antriebseinheit aufweist, sondern eine mechanische Voll- oder Teilkompensation. Bei einer mechanischen Kompensation eines Lastdrehmoments ist die Haltevorrichtung beispielsweise derart konstruiert, dass die Haltevorrichtung mittels Ausgleichsgewichten in möglichst jeder Stellung in einem Gleichgewichtszustand gehalten wird. Die Kompensation erfolgt hierbei durch die Ausgleichsgewichte und die ausgleichende Wirkung der Eigengewichtskraft der beweglichen Teile des Gelenks. Bei einer mechanischen Voll- oder Teilkompensation kann die Antriebseinheit folglich ausschließlich oder zumindest teilweise zur Erzeugung der eine Schwingung ausgleichenden Gegenschwingung genutzt werden.

Sowohl die Steuereinheit als auch die Einrichtung zur aktiven Schwingungsdämpfung können über eine interne oder externe Sensorik verfügen, beispielsweise Sensoren zum Erfassen der aktuellen Gelenkstellung, zum Erfassen des anliegenden Last-Drehmoments bzw. einer anliegenden Zug- oder Druckkraft oder zum Erfassen von Schwingungen der Haltevorrichtung, deren Messsignale die Grundlage für das jeweilige Steuersignal bzw. Ausgleichssignal bilden.

Weiterhin kann die Steuereinheit über eine externe Eingabeschnittstelle von einem Bediener gesteuert werden, der beispielsweise eine bestimmte Position des Gelenks vorgibt. Auf diese Weise dient die Antriebseinheit nicht nur zur Schwingungsdämpfung und gegebenenfalls aktiven Kompensation, sondern kann auch die Stellung des Gelenks verändern, was einen ferngesteuerten Betrieb der Haltevorrichtung ermöglicht.

Auch die Einrichtung zur aktiven Schwingungsdämpfung kann zusätzlich eine Eingabeschnittstelle aufweisen, über die von einem Bediener gewisse Eingaben gemacht werden können. So kann beispielsweise die Schwingungsdämpfung aktiviert und deaktiviert werden oder auf einen bestimmten Schwingungsbereich begrenzt werden.

Die Arretierung kann ferner wenigstens eine Anschlagfläche und wenigstens eine Gegenanschlagfläche umfassen, die zusammenwirken, um das Gelenk in seiner Arretierstellung zu halten. Um eine Arretierung in zwei zueinander entgegengesetzte Bewegungsrichtungen des Gelenks zu ermöglichen, können jedoch auch mehrere aneinander zugewandte Anschlagflächen und Gegenanschlagflächen vorgesehen sein.

Die Anschlagfläche bzw. die wenigstens eine Gegenanschlagfläche können an unterschiedlichsten Teilen der Haltevorrichtung angeordnet sein, beispielsweise jeweils an den zueinander bewegbaren Teilen des Gelenks, an dem bewegbaren Gelenkteil und einem unbeweglichen Teil der Haltevorrichtung, an der Antriebseinheit und einem unbeweglichen Teil der Haltevorrichtung etc.. Es muss nur sichergestellt sein, dass hierdurch das Gelenk in einer Arretierposition gehalten werden kann, wenn die Freigabeeinheit nicht aktiviert ist.

Zudem kann die Arretierung eine Bremse, insbesondere eine elektromagnetische Bremse, umfassen, wobei ein bewegbarer Teil der Bremse mit dem wenigstens einen angetriebenen Teil des Gelenks gekoppelt ist und wobei die wenigstens eine Anschlagfläche der Arretierung an dem bewegbaren Teil der Bremse und die wenigstens eine Gegenanschlagfläche an dem wenigstens einen angetriebenen Teil des Gelenks angeordnet sind. Statt mit dem wenigstens einen angetriebenen Teil des Gelenks kann der bewegbare Teil der Bremse selbstverständlich auch mit einem Antriebsausgang der Antriebseinheit gekoppelt sein, der mit dem angetriebenen Teil des Gelenks verbunden ist.

Eine solche Bremse ermöglicht es, das wenigstens eine angetriebene Gelenkteil zu blockieren, so dass das Gelenk mit Hilfe der Bremse in der jeweiligen Arretierposition, die es bei Aktivierung der Bremse innehatte, gehalten wird. Die kraftschlüssige Gestaltungsvariante einer Bremse, bei der der bewegbare Teil der Bremse in kraftschlüssige Anlage mit einem weiteren unbeweglichen Teil der Bremse gelangt, der mit der Haltevorrichtung oder einem unbeweglichen Teil des Gelenks gekoppelt ist, hat weiterhin den Vorteil, dass bei einer entsprechenden Auslegung der wirkenden Bremskraft das Gelenk gegebenenfalls noch von Hand bewegt werden kann (durch

Überwindung der Maximalbremskraft bzw. eines daraus resultierenden Maximalbremsmoments), um die Haltevorrichtung bei eingefallenen Bremsen und einem Ausfall der Haltevorrichtung in eine gewünschte Position bewegen zu können.

Weiterhin ist insbesondere die Ausgestaltung einer Bremse als Ruhestrombremse, z.B. als eine elektromagnetische Ruhestrombremse, von Vorteil, da diese aufgrund ihrer konstruktiven Ausgestaltung eine Bremskraft ausübt, solange keine elektrische Spannung an ihr anliegt. Auf diese Weise kann sichergestellt werden, dass die Arretierung bei einem möglichen Ausfall der Stromversorgung der Haltevorrichtung diese automatisch in einer Sicherheitsstellung halten kann, in der eine Gefährdung durch eine weitere, unerwünschte Bewegung der Haltevorrichtung ausgeschlossen ist.

Alternativ oder zusätzlich kann die Arretierung einen Anschlag aufweisen, insbesondere einen Anschlagstift, an dem die wenigstens eine Anschlagfläche ausgebildet ist und der in eine Ausnehmung mit wenigstens zwei Gegenanschlagflächen zur Arretierung der Bewegung des Gelenks in zwei Bewegungsrichtungen eingreifen kann. Diese Gestaltung entspricht einer formschlüssigen Kopplung der beweglichen Teile des Gelenks oder der Antriebseinheit mit einem nicht angetriebenen Teil des Gelenks. Bei einer Gestaltung als Anschlagstift bildet derjenige Teil der Mantelfläche des Stifts die Anschlagfläche, der bei einer Bewegung des Gelenks in blockierenden Kontakt mit einer der Gegenanschlagflächen gelangt.

Ein solcher Anschlag kann ferner zwischen einer ersten Stellung, in der er die Bewegung des Gelenks arretiert, und einer zweiten Stellung, in der er die Bewegung des Gelenks zulässt, hin und her bewegbar sein. So können die Gegenanschlagflächen als fest ausgebildete Flächen vorgesehen sein, die mit einem ein- und ausfahrbaren Anschlag oder Anschlagstift zusammenwirken. Der Anschlagstift oder Anschlag kann ferner in eine der beiden Stellungen, beispielsweise die Freigabestellung vorgespannt sein, so dass bei einer Aktivierung der Freigabeeinheit der Anschlag in der Freigabestellung gehalten und bei einer Deaktivierung der Anschlag gelöst wird und aufgrund der Vorspannung automatisch in die Arretierposition bewegt wird. Mögliche Ausgestaltungen für einen solchen Mechanismus umfassen beispielsweise eine Feder zur Vorspannung des Anschlags in einer der beiden Stellungen und einen Magneten oder dergleichen zum Bewegen und Halten des Anschlags in die bzw. in der jeweils anderen Stellung.

Ferner kann vorgesehen sein, dass zwischen der Anschlagfläche der Arretierung und der Gegenanschlagfläche in Richtung der Bewegung des Gelenks ein Spiel vorgesehen ist. Das vorgesehene Spiel dient hierbei dazu, den vorgegebenen Bewegungsbereich festzulegen, innerhalb dessen sich das Gelenk bewegen kann, wenn die Freigabeeinheit nicht aktiviert ist, d.h. die Haltevorrichtung arretiert ist.

Weiterhin kann vorgesehen sein, dass zwischen der Anschlagfläche der Arretierung und der Gegenanschlagfläche in Richtung der Bewegung des Gelenks wenigstens ein elastisches Zwischenelement vorgesehen ist. Diese Ausgestaltungsvariante lässt sich als Alternative zu dem vorgenannten Spiel vorsehen oder als zusätzliche Maßnahme. Bei einer Alternativausgestaltung ersetzt das elastische Zwischenelement das Spiel und ermöglicht aufgrund seiner Elastizität eine gewisse Bewegung des Gelenks innerhalb eines vorgegebenen Bewegungsbereichs auch dann, wenn die Freigabeeinheit nicht aktiviert ist. Der Bewegungsbereich des Gelenks ergibt sich hierbei durch die Elastizität, d.h. die Nachgiebigkeit des elastischen Zwischenelements und die auf das Zwischenelement einwirkende Kraft bzw. das einwirkende Drehmoment, die bzw. das von der Antriebseinheit eingeleitet wird.

Als ein solches elastisches Zwischenelement kann beispielsweise ein Gummielement, eine Gummibeschichtung oder dergleichen vorgesehen sein. Alternativ sind jedoch auch Ausgestaltungen des Elements aus einem anderen elastischen Material als Gummi denkbar, zum Beispiel Blattfedern oder dergleichen aus Metall. Das elastische Zwischenelement kann ferner an der Anschlagfläche, an der Gegenanschlagfläche oder an beiden angebracht sein.

Durch Vorsehen eines elastischen Zwischenelements wird zudem eine automatische Zentrierung bzw. Ausrichtung der Anschlagfläche gegenüber der Gegenanschlagfläche infolge der elastischen Rückstellkraft des elastischen Zwischenelements ermöglicht. Bei einer deaktivierten Freigabeeinheit kann die Anschlagfläche infolge einer einwirkenden Antriebskraft bzw. eines einwirkenden Drehmoments der Antriebseinheit weiterhin innerhalb des vorbestimmten Bewegungsbereichs zu der Gegenanschlagfläche hinbewegt werden. Dabei wird das elastische Zwischenelement zusammengedrückt und der Abstand zwischen der Anschlagfläche und der Gegenanschlagfläche verringert sich. Entfällt die antreibende Einwirkung der Antriebseinheit, d.h. das einwirkende Drehmoment bzw. die einwirkende Antriebskraft, oder wird sie verringert, so dass die elastische Rückstellkraft größer ist als die verbleibende antreibende Einwirkung, so wird die Anschlagfläche durch die elastische Rückstellkraft relativ zu der Gegenanschlagfläche zurückgestellt (Ausrichtung). Bei der Ausgestaltungsvariante mit einem zwischen zwei Gegenanschlagflächen angeordneten Anschlag und mit zwei spielfreien elastischen Zwischenelementen zwischen dem Anschlag und den Gegenanschlagflächen hat dies eine Zentrierung des Anschlags zwischen den Gegenanschlagflächen zur Folge.

Infolge dieser Ausrichtung bzw. Zentrierung kann sichergestellt werden, dass nach einer freien Bewegung des Gelenks (bei aktivierter Freigabeeinheit) die Anschlagfläche stets derart zu der Gegenanschlagfläche angeordnet ist, dass bei deaktivierter Freigabeeinheit das elastische Zwischenelement erneut verformt werden kann und so eine weitere Bewegung des Gelenks möglich bleibt.

Grundsätzlich ist es jedoch auch denkbar, eines oder mehrere Elemente der Haltevorrichtung derart zu gestalten, dass es oder sie eine Elastizität aufweisen, die dazu ausreicht, eine Bewegung des Gelenks in dem vorbestimmten Bewegungsbereich zuzulassen und dadurch eine Schwingungsdämpfung zu ermöglichen. Der Bewegungsbereich des Gelenks ergibt sich in diesem Fall durch die resultierende Elastizität, d.h. die resultierende Nachgiebigkeit des Systems im Bereich der Arretierung, und die auf die Arretierung einwirkende Kraft bzw. das einwirkende Drehmoment, die bzw. das von der Antriebseinheit eingeleitet wird.

Um bei der Gestaltungsvariante mit einem Anschlag zu ermöglichen, dass das Gelenk in jeder Momentanposition mit Hilfe des Anschlags arretiert werden kann, ist es vorteilhaft, eine Vielzahl von Ausnehmungen bereitzustellen, in die der Anschlag eingreifen kann.

Ferner kann die Haltevorrichtung eine Überwachungseinrichtung aufweisen, die geeignet ist, zu überwachen, ob sich das Gelenk innerhalb des vorgegebenen Bewegungsbereichs bewegt, und der Sicherungseinrichtung eine Rückmeldung hierüber zu geben. Eine solche Überwachungseinrichtung kann beispielsweise über eine Kommunikationsleitung mit der Sicherungseinrichtung verbunden sein, so dass die Rückmeldung an die Sicherungseinrichtung in Form eines Eingangssignals an die Sicherungseinrichtung weitergegeben wird.

Zudem kann die Sicherungseinrichtung basierend auf der Rückmeldung der Überwachungseinrichtung eine Aktivierung der Arretierung zulassen. Bei dieser Ausgestaltung erfolgt die Aktivierung der Arretierung quasi zweistufig: in der ersten Stufe wird durch ein Deaktivieren der Freigabeeinheit die Überwachungseinrichtung aktiviert, die überwachen soll, ob das Gelenk innerhalb des vorgegebenen Bewegungsbereichs bewegt wird. Solange dies der Fall ist, wird die Arretierung nicht aktiviert, obwohl die Freigabeeinheit deaktiviert wurde. In der zweiten Stufe gibt die Überwachungseinrichtung an, dass die Bewegung des Gelenks den vorgegebenen Bewegungsbereich verlassen hat, so dass nun von der Sicherungseinrichtung die Arretierung aktiviert wird und das Gelenk blockiert. Dies stellt sicher, dass das Gelenk keine über den beispielsweise zur Schwingungsdämpfung nutzbaren Bewegungsbereich hinausgehende Bewegung ausführen kann.

Die Überwachungseinrichtung kann zur Überwachung der Bewegung des Gelenks wenigstens einen Sensor aufweisen. Ein solcher Sensor kann beispielsweise einen Sensor oder Kodierer (Encoder) der Antriebseinheit umfassen, z.B. einen visuellen Positionssensor oder dergleichen. Besonders günstig ist es, einen Sensor zu verwenden, der bereits in der internen oder externen Sensorik der Steuereinheit oder Einheit zur aktiven Schwingungsdämpfung der Haltevorrichtung vorhanden ist und der dazu genutzt wird, eine präzise Stellung des Drehgelenks zu ermitteln.

Die vorliegende Erfindung betrifft ferner ein Verfahren zum Betreiben einer Sicherungseinrichtung für eine Haltevorrichtung mit den Merkmalen des Patentanspruchs 12.

Die Erfindung wird im Folgenden beispielhaft anhand der beiliegenden schematischen Figuren näher erläutert. Es zeigen:
- Figur 1: eine schematische Seitenansicht einer erfindungsgemäßen Haltevorrichtung,
- Figur 2: eine schematische, teilgeschnittene Seitenansicht eines Drehgelenks einer erfindungsgemäßen Haltevorrichtung gemäß Figur 1,
- Figuren 3a-e: schematische Detailansichten des Arretierungsmechanismus gemäß Figur 2, wobei diese unterschiedliche Stellungen und Ausführungsformen des Arretierungsmechanismus in perspektivischer Darstellung zeigen,
- Figuren 4a und 4b: schematische, teilgeschnittene Seitenansichten des Arretierungsmechanismus gemäß Detail A der Figur 2,
- Figur 5: eine schematische, teilgeschnittene Seitenansicht einer Bremse einer erfindungsgemäßen Haltevorrichtung gemäß Figur 1,
- Figur 6: eine schematische Detailansicht der Kopplung der Bremse gemäß Figur 5 mit einem Teil des Gelenks der erfindungsgemäßen Haltevorrichtung, und
- Figur 7: ein Flussdiagramm, das die Vorgänge innerhalb einer Sicherungseinrichtung der erfindungsgemäßen Haltevorrichtung beschreibt.

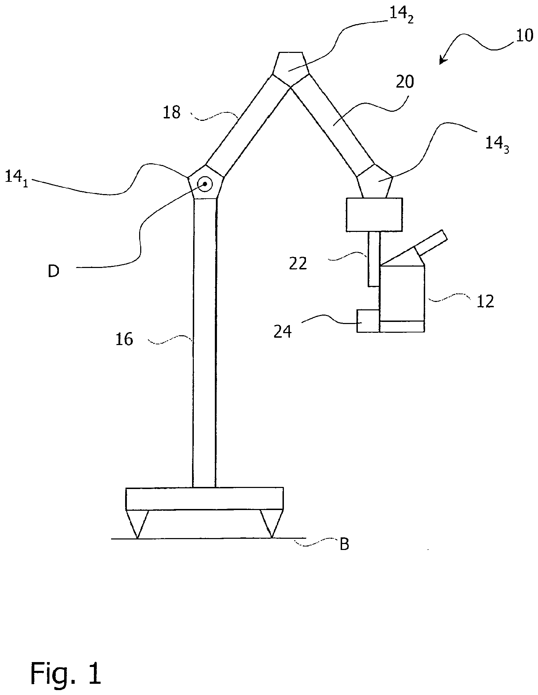

In Figur 1 ist eine stark vereinfacht dargestellte, beispielhafte Ausführungsform einer erfindungsgemäßen Haltevorrichtung in Seitenansicht gezeigt. Die Haltevorrichtung ist hierbei allgemein mit dem Bezugszeichen 10 bezeichnet. Die Vorrichtung 10 umfasst ein Instrument 12, beispielsweise wie dargestellt ein Mikroskop oder dergleichen, das mit der Haltevorrichtung 10 verbunden ist. Die Haltevorrichtung 10 ist in der dargestellten Ausführungsform der Figur 1 am Boden B eines Raumes angeordnet. Alternativ könnte die Haltevorrichtung 10 jedoch selbstverständlich auch an den Seitenwänden oder einer Deckenwand eines Raums befestigt sein.

Die dargestellte Haltevorrichtung 10 ist als ein sogenanntes Stativ ausgeführt, das mehrere starre Elemente 16, 18, 20 (nachfolgend als "Arme" bezeichnet) aufweist, die durch mehrere Drehgelenke 14 miteinander verbunden sind. So sind die Arme 16 und 18 durch das Drehgelenk 14₁ relativ zueinander bewegbar miteinander verbunden und die Arme 20 und 18 durch das Drehgelenk 14₂. An dem freien Ende der Haltevorrichtung 10 ist eine Instrumentaufnahme 22 angebracht, die ihrerseits über ein Drehgelenk 14₃ mit dem Arm 20 verbunden ist. Je nach Art und Ausbildung der Haltevorrichtung 10 kann das Instrument 12 auf diese Weise um mehrere Achsen bewegt werden, welche parallel oder quer zueinander angeordnet sein können. Alternativ zu der gezeigten Haltevorrichtung 10 umfasst die vorliegende Erfindung selbstverständlich auch Haltevorrichtungen, die eines oder mehrere Schubgelenke anstelle eines oder zusätzlich zu einem Drehgelenk umfassen. Beliebige Gelenkkombinationen mit unterschiedlich vielen und unterschiedlich ausgebildeten Gelenken sind hierbei möglich.

Die nachfolgenden Ausführungen zur Funktionsweise und Ausgestaltung des Drehgelenks 14₁ gelten ebenfalls für die Drehgelenke 14₂ und 14₃.

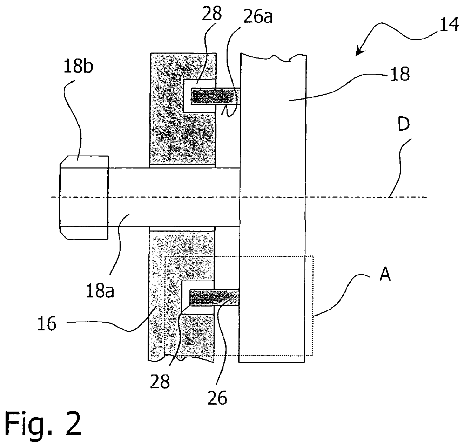

Den einzelnen Drehgelenken 14 der Haltevorrichtung 10 sind Antriebseinheiten (nicht dargestellt) zugeordnet, beispielsweise in der Form eines Elektromotors oder dergleichen, welche dazu dienen, zumindest eines der beweglichen Teile des Drehgelenks 14 anzutreiben und auf diese Weise relativ zu dem anderen Teil zu bewegen. So wird, wie in Figur 2 gezeigt, der Arm 18 relativ zu dem Arm 16 bewegt, indem eine mit dem Arm 18 verbundene Antriebswelle 18a an ihrem freien Ende eine Schnittstelle 18b aufweist, über die sie mit der Antriebseinheit derart verbunden werden kann, dass sich die Antriebswelle 18a zusammen mit dem Arm 18 von der Antriebseinheit angetrieben um die Drehachse D bewegen kann.

Die Antriebswelle 18a erstreckt sich bei der in Figur 2 gezeigten Ausführungsform durch eine Durchgangsbohrung des Arms 16, wobei die Welle 18a innerhalb der Durchgangsbohrung reibungsarm gelagert sein kann (nicht dargestellt). Die in Figur 2 dargestellte Ausgestaltung des Drehgelenks 14 ist jedoch nur exemplarisch; so kann die Anbindung 18b an eine Antriebseinheit auch an einer anderen Stelle und in einer vollkommen anderen Weise gestaltet sein, wie beispielsweise mit einer in den Arm 18 eingreifenden Antriebswelle der Antriebseinheit, einer an der Außenkontur des Arms 18 angreifenden Antriebseinheit und dergleichen.

Üblicherweise ist bei einer Haltevorrichtung, wie der erfindungsgemäßen Haltevorrichtung 10, der Antrieb bzw. die Antriebseinheit an jedem Drehgelenk 14 vorgesehen. Diese kann dazu dienen, ein Last-Drehmoment auszugleichen, welches bedingt durch das Eigengewicht des an der Haltevorrichtung 10 angebrachten Instruments 12 und der einzelnen Elemente der Haltevorrichtung in dem jeweiligen Drehgelenk 14 erzeugt wird. So wird beispielsweise an dem Drehgelenk 14₁ ein Last-Drehmoment erzeugt, das aus den Gewichtskräften aller mit dem Arm 18 verbundenen Elemente (einschließlich des Instruments 12) und dem zugehörigen Hebelarm resultiert.

Um eine möglichst einfache manuelle Handhabung (manuelle Positionierung) des Instruments 12 gewährleisten zu können, dienen die Antriebseinheiten in den jeweiligen Drehgelenken 14 dazu, das jeweilige Last-Drehmoment durch ein gleichgroßes Gegenmoment auszugleichen, so dass bei einer manuellen Bewegung des Instruments 12 im Arbeitsraum der Haltevorrichtung 10 diese quasi schwerelos von einem Bediener bewegt werden kann sowie Trägheiten kompensiert werden (aktive Kompensation). Allenfalls sind von dem Bediener, der das Instrument 12 frei bewegen möchte, die in den Drehgelenken 14 auftretenden Reibungskräfte zu überwinden, welche (bei gelöster Arretierung) gering sind und je nach Gestaltung der Haltevorrichtung ebenfalls - wie auch auftretende Trägheiten - durch die Antriebeinheit kompensiert werden können (aktive Kompensation). Alternativ kann die erfindungsgemäße Haltevorrichtung jedoch auch eine mechanische Voll- oder Teilkompensation auftretender Last-Drehmomente in jedem Gelenk aufweisen.

Wie in Figur 2 ebenfalls zu erkennen ist, weist das Drehgelenk 14₁ (ebenso wie die Drehgelenke 14₂ und 14₃) zudem eine Arretierung, z.B. in Form eines Anschlagstiftes 26, auf, die dazu dient, die zueinander beweglichen Teile des Drehgelenks 14₁ miteinander zu koppeln, um so eine Drehbewegung des Drehgelenks 14₁ zu blockieren. Mit Hilfe der Arretierung kann die Haltevorrichtung 10 nach Abschluss der Positionierung die gewünschte Position beibehalten. Alternativ zu einer in Figur 2 gezeigten, formschlüssigen Kopplung der zueinander beweglichen Teile 16, 18 des Drehgelenks 14₁ ist auch eine kraftschlüssige Kopplung denkbar, wie nachfolgend ausgeführt. Zudem ist es gleichermaßen möglich, die Arretierung zwischen der Antriebseinheit und dem nicht von einer Antriebseinheit angetriebenen Teil 16 des Drehgelenks 14₁ oder zwischen dem angetriebenen Teil 18 des Drehgelenks 14₁ und einem unbeweglichen Teil der Haltevorrichtung 10 anzuordnen, um diese Elemente miteinander zu koppeln.

Eine an dem Instrument 12 angeordnete Freigabeeinheit 24 bildet zusammen mit der Arretierung eine sogenannte Sicherungseinrichtung, die dazu dient, im Arbeitsraum der Haltevorrichtung 10 befindliche Menschen vor unbeabsichtigten Bewegungen der Haltevorrichtung 10 zu schützen. So kann die Haltevorrichtung 10 nur dann frei in ihrem gesamten Arbeitsraum bewegt werden, wenn die Freigabeeinheit 24 aktiviert ist, wobei sie in ihrem aktivierten Zustand derart mit der Arretierung zusammenwirkt, dass letztere das Drehgelenk 14 freigibt. Sobald die Freigabeeinheit 24 nicht aktiviert ist, soll eine derartige freie Bewegbarkeit der Haltevorrichtung 10 in ihrem Arbeitsraum aus Sicherheitsgründen nicht mehr möglich sein.

Die Freigabeeinheit 24 kann einen Handgriff, einen Druckknopf, einen Joystick oder jede andere Art der Eingabeeinheit umfassen, die der Benutzer betätigen oder aktivieren kann, um auszudrücken, dass er die Haltevorrichtung 10 bewegen möchte.

Aufgrund ihres Aufbaus bietet die erfindungsgemäße Haltevorrichtung 10 neben der vorstehend geschilderten manuellen Bewegbarkeit auch die Möglichkeit, automatisch mittels einer Steuerung bewegt zu werden. Grundsätzlich erzeugt eine nicht dargestellte Steuereinheit beispielsweise zum Aufbau eines Gegendrehmoments in dem jeweiligen Drehgelenk 14 ein Steuersignal, welches bewirkt, dass die Antriebseinheit wenigstens eines der zueinander beweglichen Teile des Drehgelenks 14 wie gewünscht antreibt und auf diese Weise das Drehgelenk 14 bewegt. Ist die Freigabeeinheit 24 aktiviert, kann die Haltevorrichtung 10 jedoch auch mittels der selben oder einer zusätzlichen Steuereinheit in eine gewünschte Position bewegt werden, indem die Steuereinheit die entsprechenden Antriebe der Drehgelenke 14 ansteuert (ferngesteuerter Betrieb).

Weiterhin ist die Haltevorrichtung 10 derart gestaltet, dass auftretende Schwingungen, beispielsweise bedingt durch ein Nachschwingen der Haltevorrichtung 10 nach einer erfolgten Positionierungsbewegung (manuell oder automatisch) oder bedingt durch Raumschwingungen, die beispielsweise über den Boden B auf die Haltevorrichtung 10 übertragen werden, gedämpft werden. Dies wird über eine Einrichtung zur aktiven Schwingungsdämpfung (nicht dargestellt) erreicht, die die Antriebseinheiten der angetriebenen Drehgelenke 14 zur Schwingungsdämpfung verwendet. Hierzu gibt die Einrichtung zur aktiven Schwingungsdämpfung ein (ggf. das Steuersignal überlagerndes) Ausgleichsignal an die jeweilige Antriebseinheit ab, welches bewirkt, dass die Antriebseinheit das Drehgelenk 14 so bewegt, dass Schwingungen der Haltevorrichtung 10 kompensiert werden. Üblicherweise ist ein solches Ausgleichsignal derart gestaltet, dass es eine periodische Bewegung des Drehgelenks 14 (Anregerschwingung) entsprechend der wirkenden Schwingung bewirkt, wobei durch einen Phasenversatz der Anregerschwingung zur wirkenden Schwingung die wirkende Schwingung und die Anregerschwingung sich gegenseitig auslöschen.

Damit eine solche aktive Schwingungsdämpfung auch dann noch möglich ist, wenn die Haltevorrichtung nicht frei im Arbeitsraum bewegbar ist, sondern in einer Haltestellung verbleiben soll, ist die Sicherungseinrichtung dazu eingerichtet, eine Bewegung des Drehgelenks 14 innerhalb eines vorgegebenen Winkelbereichs aus der jeweiligen Arretierposition heraus zuzulassen, wenn die Freigabeeinheit 24 nicht aktiviert ist.

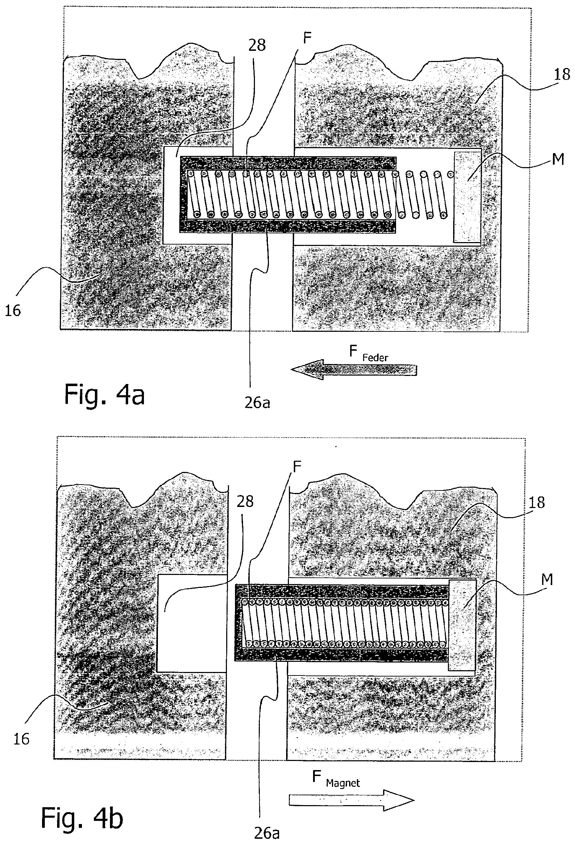

In einer ersten Ausgestaltungsvariante umfasst die Sicherungseinrichtung, wie in Figuren 2 bis 4b gezeigt, eine Arretierung mit wenigstens einem Anschlagstift 26 (in den Figuren 4a und b sind zwei Anschlagstifte 26 gezeigt), dessen Außenumfangsfläche eine Anschlagfläche 26a bildet. Wie in den Figuren 4a und 4b dargestellt, kann jeder Anschlagstift 26 zwischen einer ersten Stellung, in der er die Bewegung des Drehgelenks 14 arretiert, und einer zweiten Stellung in der er die Bewegung des Drehgelenks zulässt, hin und her bewegt werden. Jeder Anschlagstift 26 ist in seiner ersten Stellung in einer Ausnehmung 28 (in den Figuren 4a und b sind zwei Ausnehmungen 28 gezeigt) aufgenommen, welche Gegenanschlagflächen 28a und 28b aufweist. Jeder Anschlagstift 26 kann an dem von der Antriebseinheit angetriebenen Teil 18 des Drehgelenks 14₁ oder an dem nicht angetriebenen Teil 16 des Drehgelenks 14₁ angeordnet sein, wobei die Ausnehmungen 28, welche die Gegenanschlagflächen 28a und 28b bereitstellen, an dem jeweils anderen Teil des Drehgelenks 14₁ angeordnet sind.

Der gezeigte Mechanismus zum Hin- und Herbewegen der Anschlagstifte 26 umfasst gemäß den Figuren 4a und 4b einen Magneten M sowie eine Feder F. Die Feder F ist in dem Inneren des Anschlagstifts 26 aufgenommen und spannt diesen in einer solchen Weise vor, dass er sich in der ersten arretierenden Stellung befindet, wenn die Feder F entspannt ist. Dies ist auch durch den Pfeil F_{Feder} der Figur 4a angedeutet. Selbstverständlich sind auch andere Anordnungsmöglichkeiten der Feder F denkbar.

Der Magnet M (der schematisch eine wie auch immer geartete Quelle magnetischer Anziehungskraft darstellen soll) dient dazu, den Anschlagstift 26 in dessen zweite Stellung (vergleiche Figur 4b) zu verlagern, in der er die Bewegung des Drehgelenks 14 zulässt. Hierzu wirkt eine durch den Pfeil F_{Magnet} symbolisierte magnetische Kraft wie in Figur 4b gezeigt auf den Anschlagstift 26, wodurch die Feder F vorgespannt wird. Bei dem Magneten M handelt es sich nicht um einen Permanentmagneten, sondern um einen elektrisch aktivierbaren Magneten, beispielsweise in der Form einer Spule oder dergleichen. Wird der Magnet M nicht mit Strom beaufschlagt, so wirkt keine magnetische Kraft F_{Magnet} auf den Anschlagstift 26, so dass dieser infolge der Federkraft F_{Feder} der vorgespannten Feder F wieder in seine erste Stellung (vergleiche Figur 4a) zurück bewegt wird.

Der gezeigte Mechanismus zur Bewegung des Anschlags ist rein exemplarisch und kann beispielsweise durch Wirkungsumkehr oder Verwendung anderer Elemente zur Krafteinleitung ersetzt werden.

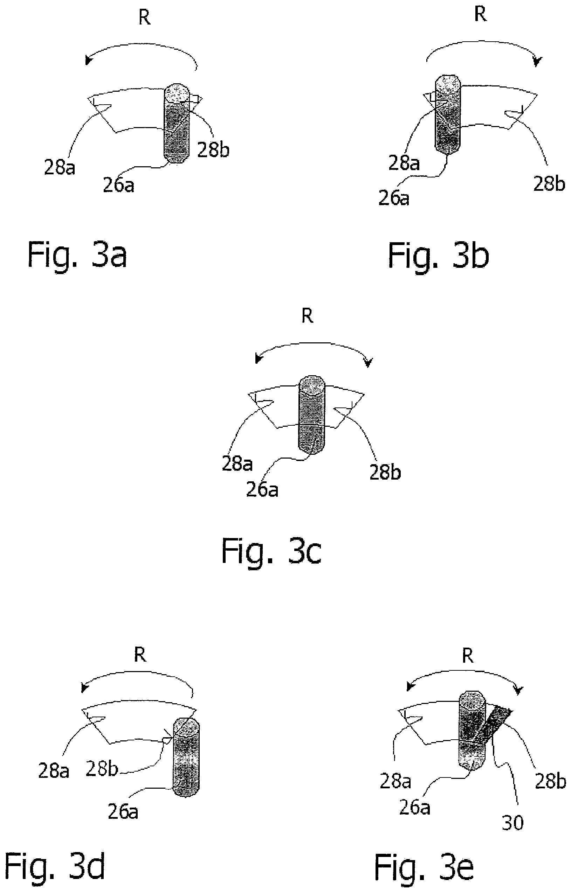

In den Figuren 3a bis 3c ist eine mögliche Ausgestaltung der Ausnehmungen 28 mit den Gegenanschlagflächen 28a und 28b gezeigt, in der ein Anschlagstift 26 in unterschiedlichen Positionen relativ zu den Gegenanschlagflächen 28a, 28b aufgenommen ist. So ist die Ausnehmung 28, wie deutlich erkennbar, derart ausgestaltet, dass zwischen der Anschlagfläche 26a des Anschlagstifts 26 und der Gegenanschlagfläche 28a bzw. 28b in Richtung der Bewegung des Drehgelenks 14 (angedeutet durch den Pfeil R) ein Spiel vorgesehen ist, so dass eine geringfügige Bewegung des Drehgelenks 14₁ innerhalb eines vorgegebenen Winkelbereichs auch dann möglich ist, wenn die Arretierung aktiviert ist.

Um in möglichst jeder denkbaren Position eine Arretierung des Drehgelenks 14 ermöglichen zu können, ist es notwendig, eine Vielzahl von Ausnehmungen 28 mit entsprechenden Gegenanschlagflächen 28a, 28b vorzusehen sowie wenigstens zwei Anschlagstifte 26, die zueinander und bezüglich der Gegenanschlagflächen 28a, 28b derart mit Winkelversatz (zu der Rotationsachse D) angeordnet sind, dass zumindest immer einer der Anschlagstifte 26 in eine Ausnehmung 28 eingreifen kann, selbst wenn der andere durch eine Gegenanschlagfläche 28b blockiert ist (vergleiche Figur 3d).

Weiterhin ist die Ausgestaltung des Anschlagstifts 26 als ein Kreiszylinder gemäß den Figuren 2 bis 4b und der Ausnehmung gemäß den Figuren 3a bis e rein exemplarisch. So kann bei einer alternativen Ausgestaltung, beispielsweise mit kegelförmigen oder sägezahnförmigen Anschlägen und korrespondierenden Ausnehmungsflächen, ein unerwünschtes Blockieren der Bewegung des Anschlagstifts 26 aus der zweiten in seine erste Stellung durch eine Gegenanschlagfläche 28b (wie in Figur 3d gezeigt) weitestgehend vermieden werden.

Zudem ist es möglich, anstelle oder in Kombination mit dem vorstehend diskutierten Spiel in Richtung R der Bewegung des Drehgelenks 14₁ ein elastisches Zwischenelement E zwischen der Anschlagfläche des Anschlags und der Gegenanschlagfläche 28a, 28b (wie in Figur 3e gezeigt) vorzusehen, das auch dann eine weitere Bewegung des Drehgelenks 14₁ zulässt, wenn der Anschlag bereits an dem elastischen Zwischenelement E anliegt (vergleiche Figur 3e).

Das elastische Zwischenelement E kann sowohl an der Gegenanschlagfläche 28a, 28b oder an der Anschlagfläche 26a angeordnet sein. Beispielsweise ist es denkbar, den Anschlagstift 26 oder einen wie auch immer gearteten Anschlag mit einer Ummantelung aus einem elastischen Material, beispielsweise Gummi, zu versehen.

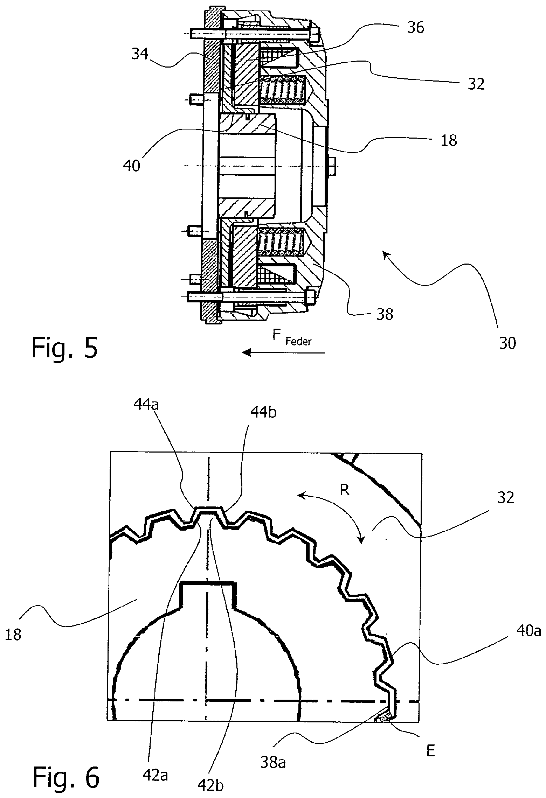

Eine alternative, kraftschlüssige Art der Arretierung kann eine Bremse 30 (vgl. Figuren 5 und 6) umfassen, die entweder die zueinander beweglichen Teile 16, 18 des Drehgelenks 14 oder die Antriebseinheit mit einem unbeweglichen Teil, beispielsweise dem nicht durch die Antriebseinheit angetriebenen Arm (in Figur 2 der Arm 16), miteinander koppelt. Ein Beispiel für solch eine Bremse 30 ist eine sogenannte elektromagnetische Ruhestrombremse, die eine unter Vorspannung gehaltene Bremsscheibe 32 aufweist, welche gegen eine korrespondierende Bremsanlagefläche 34 drückt und so den Kraftschluss herstellt. Hierzu presst eine Feder F mit einer Druckscheibe 36 die Bremsscheibe 32 gegen die korrespondierende Bremsanlagefläche 34. Erst wenn eine elektrische Spannung an die Ruhestrombremse angelegt wird, wird die Druckscheibe 36 durch magnetische Kräfte (die von einem bestromten Spulenträger 38 erzeugt werden) von der Bremsanlagefläche 34 weg und entgegen einer Vorspannkraft F_{Feder} der Feder F bewegt, so dass ein axialer Abstand zwischen der Bremsanlagefläche 34 und der Bremsscheibe 32 der Ruhestrombremse entsteht. In dieser Stellung ist der Kraftschluss aufgehoben und die zuvor gebremsten Teile können frei relativ zueinander bewegt werden.

Der Vorteil einer solchen Ruhestrombremse besteht insbesondere darin, dass bei einem möglichen Stromausfall die Bremse automatisch aktiviert wird und so verhindert werden kann, dass sich die Haltevorrichtung infolge ihres Eigengewichts in unerwünschter Weise bewegt. Ein weiterer Vorteil einer kraftschlüssigen Ausgestaltung der Arretierung, wie beispielsweise in Form einer Bremse, besteht darin, dass sich die zueinander beweglichen Teile notfalls durch Überwindung der Reibungskräfte der eingefallenen Ruhestrombremse auch dann noch bewegen lassen, wenn die Arretierung aktiv ist, was es bei einem Systemausfall ermöglicht, die Haltevorrichtung manuell aus einer potenziellen Gefahrenstellung heraus zu bewegen.

Bei der in den Figuren 5 und 6 gezeigten Bremse 30 ist die Bremsscheibe 32 mit dem angetriebenen Teil 18 des Drehgelenks 14₁ gekoppelt, während die Bremsanlagefläche 34 beispielsweise starr mit dem weiteren Teil 16 des Drehgelenks 14₁ oder mit einem unbeweglichen Teil der Haltevorrichtung 10 verbunden sein kann. Die Kopplung der Bremsscheibe 32 mit dem angetriebenen Teil 18 erfolgt über eine keilwellenartige Verzahnung 38a an der Außenumfangsfläche einer Antriebswelle 18a des angetriebenen Teils 18 mit einer im wesentlichen korrespondierenden Verzahnung 40a an der Innenumfangsfläche einer zentralen Ausnehmung 40 der Bremsscheibe 32. Jeder Zahn der Verzahnung 38a weist zwei seitliche Flanken 42a, 42b auf, die die Anschlagflächen der erfindungsgemäßen Arretierung bilden, wobei die im wesentlichen korrespondierenden Ausnehmungen an der Verzahnung 40a jeweils zwei Flanken 44a, 44b aufweisen, die die Gegenanschlagflächen der erfindungsgemäßen Arretierung bilden. Zwischen jeder Anschlagfläche und jeder korrespondierenden Gegenanschlagfläche ist in Bewegungsrichtung R des Teils 18 ein Spiel und/oder ein elastisches Element E (in Figur 6 ist nur eines beispielhaft dargestellt) vorgesehen, wie auch in Zusammenhang mit dem Anschlagstift 26 vorstehend unter Bezugnahme auf die Figuren 3a bis 3c und 3e erläutert. Das elastische Element E dient dabei zur Selbstzentrierung der Bremsscheibe 32 relativ zu der über die ineinandergreifenden Verzahnungen 38a und 40a angebundenen Antriebswelle 18a des angetriebenen Teils 18.

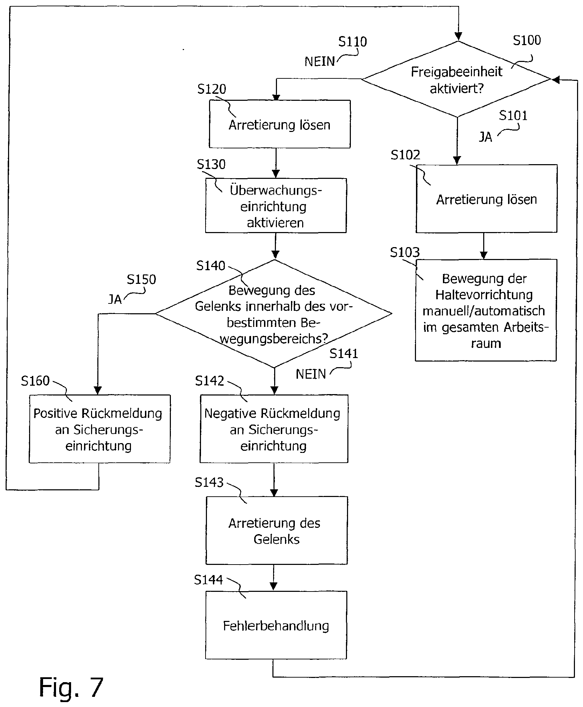

In Figur 7 schließlich ist ein Flussdiagramm gezeigt, das die Wirkungsweise der Sicherungseinrichtung einer erfindungsgemäßen Haltevorrichtung 10 beispielhaft darstellt. Bei dieser Ausführungsform ist zusätzlich oder alternativ zu der in den Figuren 2 bis 6 gezeigten Gestaltung der Arretierung eine software-basierte Steuerung der Sicherungseinrichtung vorgesehen.

In einem Schritt S100 überprüft eine Steuerung der Sicherungseinrichtung, ob die Freigabeeinheit 24 aktiviert ist. Hierzu kann beispielsweise ein üblicher Taster oder Sensor jeder Art verwendet werden, der an einem Haltegriff angeordnet, in einem Druckknopf integriert oder an einer anderen Art der Eingabeeinheit angeordnet ist und die Absicht eines Benutzers erkennen lässt, die Haltevorrichtung 10 zu bewegen. Dieser Taster oder Sensor gibt bei Aktivierung und/oder Deaktivierung der Freigabeeinheit 24, beispielsweise durch Druck auf den entsprechenden Druckknopf, ein Signal an die Steuerung der Sicherungseinrichtung ab. Erhält die Steuerung der Sicherungseinrichtung in einem Schritt S101 das Signal, dass die Freigabeeinheit 24 aktiviert ist, so gibt sie an die Arretierung ein Lösesignal aus, wodurch die Arretierung gelöst wird (Schritt S102). Voraussetzung hierfür ist selbstverständlich, dass die Arretierung, in welcher Weise auch immer, durch die Steuerung ansteuerbar ist. Eine solche Ansteuerung ist beispielsweise bei der in den Figuren 4a und 4b gezeigten Ausführungsform möglich, bei der zum Lösen der Arretierung der Magnet M mit Strom beaufschlagt wird, was den Anschlagstift 26 in seine zweite Stellung (Lösestellung) bewegt.

Hiernach ist im Schritt S103 die Haltevorrichtung manuell oder automatisch im gesamten Arbeitsraum der Haltevorrichtung 10 frei bewegbar. Wird die Freigabeeinheit 24 nach Abschluss der gewünschten Bewegung der Haltevorrichtung 10 deaktiviert, d.h. erhält die Sicherungseinrichtung in einem Schritt S110 das Signal, dass die Freigabeeinheit 24 nicht aktiviert ist, so gibt sie in einem Schritt S120 ebenfalls das Signal, zum Lösen der Arretierung (wie vorstehend unter Schritt S102 beschrieben), aktiviert jedoch in einem Schritt S130 zusätzlich eine Überwachungseinrichtung (nicht dargestellt), welche die Bewegung des wenigstens einen Drehgelenks 14 der Haltevorrichtung 10 überwacht. Die nachfolgenden Ausführungen sind auf ein Drehgelenk 14 bezogen, gelten jedoch bei einer entsprechenden Ausgestaltung der Haltevorrichtung selbstverständlich auch für mehrere Drehgelenke, wie die Drehgelenke 14₁, 14₂ und 14₃ der Haltevorrichtung 10, oder für eines oder mehrere Schubgelenke.

Die Überwachungseinrichtung gemäß der vorliegenden Erfindung kann beispielsweise eine interne oder externe Sensorik, d.h. z.B. Winkelsensoren als interne Sensoren des Drehgelenks 14 (bzw. Wegmessungssensoren für ein Schubgelenk) oder externe Sensoren einer Navigationssensorik (nicht dargestellt) etc., verwenden, um eine genau Angabe darüber zu erhalten, in welcher Stellung sich das Drehgelenk 14 (IST-Stellung) befindet und um welchen Winkel (bzw. Weg für das Schubgelenk) sich das Gelenk 14 gegebenenfalls aus der jeweiligen Arretierposition bewegt hat (Bewegungs-Winkel bzw. Bewegungs-Weg).

Hierzu kann die Überwachungseinrichtung beispielsweise diejenige Stellung des Gelenks 14 als Arretierposition speichern, in der sich das Gelenk 14 zu dem Zeitpunkt befunden hat, zu dem die Sicherungseinrichtung das Signal erhielt, dass die Freigabeeinheit 24 nicht aktiviert ist (Schritt S110). Ausgehend von dieser Arretierposition kann die Überwachungseinrichtung dann überprüfen, ob das Gelenk 14 innerhalb eines vorgegebenen Bewegungsbereichs aus dieser Arretierposition heraus bewegt wird. Hierzu kann die Überwachungseinrichtung beispielsweise anhand der Arretierposition und dem vorgegebenen Bewegungsbereich eine SOLL-Stellung berechnen und diese mit der IST-Stellung vergleichen oder den anhand der IST-Stellung ermittelbaren Bewegungs-Winkel (bzw. Bewegungs-Weg) mit einem die SOLL-Stellung charakterisierenden Winkel (bzw. Weg) vergleichen (Schritt S140).

Dabei kann der vorgegebene Winkel bzw. Weg oder der Bewegungsbereich je nach Anwendungsfall durch einen Bediener eingestellt bzw. geändert werden oder einen festen Sicherheitswert umfassen. Kommt die Überwachungseinrichtung bei einem Vergleich der IST-Stellung des Gelenks bzw. des Bewegungs-Winkels des Drehgelenks 14 (bzw. Bewegungs-Wegs des Schubgelenks) gegenüber einer SOLL-Stellung bzw. dem vorgegebenen Winkel (Weg) zu dem Ergebnis, dass das Gelenk 14 innerhalb des vorgegebenen Bewegungsbereichs bewegt wird (Schritt S150), so gibt sie eine positive Rückmeldung an die Sicherungseinrichtung (Schritt S160).

Es wird dann erneut überprüft, ob die Freigabeeinheit 24 aktiviert ist (Schritt S100), und bei weiterhin deaktivierter Freigabeeinheit 24 bleibt die Arretierung gelöst (Schritt S120) und die Überwachungseinrichtung aktiviert (Schritt S130). Erkennt die Überwachungseinrichtung jedoch in einem Schritt S140, dass die Bewegung des Gelenks 14 den vorgegebenen Bewegungsbereich zu verlassen droht (Schritt S141), so gibt sie eine negative Rückmeldung an die Sicherungseinrichtung (Schritt S142). Diese veranlasst dann in einem Schritt S143 ein Blockieren des Gelenks 14, indem sie an die Arretierung ein entsprechendes Signal ausgibt. Zudem wird eine Fehlerbehandlung (S144) angestoßen, im Rahmen derer überprüft wird, weshalb sich das Gelenk 14 über den vorgegebenen Bewegungsbereich hinaus bewegt (S144).

Die in Figur 7 beschriebene Funktionsweise der Sicherungseinrichtung kann selbstverständlich mit der mechanischen Ausgestaltung der Arretierung gemäß den Figuren 2 bis 6 kombiniert werden, wobei gegebenenfalls das Spiel zwischen der Anschlagfläche und der Gegenanschlagfläche der Arretierung derart gewählt wird, dass eine Blockierung erst dann auftritt, wenn der vorgegebene Bewegungsbereich für eine Bewegung des Gelenks verlassen wurde. In diesem Fall dient die konstruktive Ausgestaltung als zusätzliche Absicherung, falls die Steuerung der Sicherungseinrichtung ausfallen sollte.

Die erfindungsgemäße Haltevorrichtung ermöglicht durch ihre Gestaltung, insbesondere durch die Gestaltung der Sicherungseinrichtung, dass einerseits die Sicherungseinrichtung der Haltevorrichtung weiterhin den Schutz der sich innerhalb des Arbeitsraums der Haltevorrichtung befindlichen Menschen sicherstellen kann und dass andererseits auch in einer Halteposition der Haltevorrichtung eine aktive Schwingungsdämpfung unter Nutzung der bereits vorhandenen Antriebseinheiten der Gelenke stattfinden kann.

## Patentansprüche

1. Haltevorrichtung (10) für ein Instrument (12), die wenigstens ein Gelenk (14) mit wenigstens zwei relativ zueinander beweglichen Teilen (16, 18) und wenigstens eine Antriebseinheit aufweist, und die ferner eine Sicherungseinrichtung mit einer dem Gelenk (14) zugeordneten Arretierung und mit einer wahlweise aktivierbaren Freigabeeinheit (24) aufweist, wobei die Arretierung das Gelenk (14) in einer Arretierposition hält, solange die Freigabeeinheit (24) nicht aktiviert ist,
**dadurch gekennzeichnet, dass**
- die Antriebseinheit dem Gelenk (14) zugeordnet und dazu ausgebildet ist, das Gelenk (14) durch Antreiben wenigstens eines der zueinander beweglichen Teile (18) des Gelenks (14) zu bewegen, und dass
- die Sicherungseinrichtung eine Bewegung des Gelenks (14) aus der jeweiligen Arretierposition heraus innerhalb eines vorbestimmten, begrenzten Bewegungsbereichs auch dann gestattet, wenn die Freigabeeinheit (24) nicht aktiviert ist.

2. Haltevorrichtung nach Anspruch 1,
wobei Haltevorrichtung (10) ferner eine Einrichtung zur aktiven Schwingungsdämpfung aufweist, die dazu ausgebildet ist, ein Ausgleichssignal an die Antriebseinheit auszugeben, welches bewirkt, dass die Antriebseinheit das Gelenk (14) derart bewegt, dass Schwingungen der Haltevorrichtung (10) ausgeglichen werden können.

3. Haltevorrichtung nach Anspruch 1 oder 2,
wobei die Arretierung wenigstens eine Anschlagfläche (26a; 42a, 42b) sowie wenigstens eine Gegenanschlagfläche (28a, 28b; 44a, 44b) umfasst, die zusammenwirken können, um das Gelenk (14) in der Arretierposition zu halten.

4. Haltevorrichtung nach einem der Ansprüche 1 bis 3,
wobei die Arretierung eine Bremse (30), insbesondere eine elektromagnetische Bremse, mit einem bewegbaren Teil (32) umfasst, der mit dem wenigstens einen angetriebenen Teil (18) des Gelenks (14) gekoppelt ist, und wobei die wenigstens eine Anschlagfläche (42a, 42b) der Arretierung an dem bewegbaren Teil (32) der Bremse (30) und die wenigstens eine Gegenanschlagfläche (44a, 44b) an dem wenigstens einen angetriebenen Teil (18) des Gelenks (14) angeordnet ist.

5. Haltevorrichtung nach einem der Ansprüche 1 bis 4,
wobei die Arretierung einen Anschlag, insbesondere einen Anschlagstift (26), aufweist, an dem die wenigstens eine Anschlagfläche (26a) ausgebildet ist und der in eine Ausnehmung (28) mit wenigstens zwei Gegenanschlagflächen (28a, 28b) zur Arretierung der Bewegung des Gelenks in zwei entgegengesetzten Bewegungsrichtungen (R) eingreifen kann.

6. Haltevorrichtung nach Anspruch 5,
wobei der Anschlag zwischen einer ersten Stellung, in der er die Bewegung des Gelenks (14) arretiert, und einer zweiten Stellung, in der er die Bewegung des Gelenks (14) zulässt, hin und her bewegbar ist.

7. Haltevorrichtung nach einem der Ansprüche 3 bis 6,
wobei zwischen der Anschlagfläche (26a; 42) und der Gegenanschlagfläche (28a, 28b; 44a, 44b) der Arretierung in Richtung (R) der Bewegung des Gelenks (14) ein Spiel vorgesehen ist.

8. Haltevorrichtung nach einem der Ansprüche 3 bis 7,
wobei zwischen der Anschlagfläche und der Gegenanschlagfläche (28a, 28b) der Arretierung in Richtung (R) der Bewegung des Gelenks (14) wenigstens ein elastisches Zwischenelement (E) vorgesehen ist.

9. Haltevorrichtung nach einem der Ansprüche 1 bis 8,
wobei die Haltevorrichtung (10) ferner eine Überwachungseinrichtung aufweist, die dazu ausgebildet ist, zu überwachen, ob das Gelenk (14) innerhalb des vorgegebenen Bewegungsbereichs bewegt wird, und der Sicherungseinrichtung eine Rückmeldung hierüber zu geben.

10. Haltevorrichtung nach Anspruch 9,
wobei die Sicherungseinrichtung basierend auf der Rückmeldung der Überwachungseinrichtung eine Aktivierung der Arretierung zulassen kann.

11. Haltevorrichtung nach einem der Ansprüche 9 oder 10,
wobei die Überwachungseinrichtung zur Überwachung der Bewegung des Gelenks wenigstens einen Sensor aufweist.

12. Verfahren zum Betreiben einer Sicherungseinrichtung einer Haltevorrichtung (10) für ein Instrument (12), wobei die Haltevorrichtung (10) aufweist:
- mindestens ein Gelenk (14) mit wenigstens zwei relativ zueinander beweglichen Teilen (16, 18) und wenigstens eine dem Gelenk (14) zugeordnete Antriebseinheit, wobei die Antriebseinheit dazu ausgebildet ist, das Gelenk (14) zu bewegen, indem sie wenigstens eines der zueinander beweglichen Teile (18) des Gelenks (14) antreibt, und
- die Sicherungseinrichtung mit einer dem Gelenk (14) zugeordneten Arretierung und einer aktivierbaren Freigabeeinheit (24),
wobei das Verfahren die folgenden Schritte umfasst:
- Überprüfen (S100), ob die Freigabeeinheit (24) aktiviert ist,
- Lösen der Arretierung (S120),
- Aktivieren einer Überwachungseinrichtung (S130),
- Speichern, wenn die Freigabeeinheit (24) nicht aktiviert ist (S110), einer Arretierposition und Überwachen (S140) einer Bewegung des Gelenks (14) daraufhin, ob das Gelenk (14) innerhalb eines vorgegebenen Bewegungsbereichs ausgehend von dieser Arretierposition bewegt wird,
- Aktivieren der Arretierung (S143), wenn das Gelenk (14) sich über den vorgegebenen Bewegungsbereich hinaus bewegt.

## Claims

1. Retaining device (10) for an instrument (12), comprising at least one joint (14), with at least two parts (16, 18) which are movable relative to one another, and at least one drive unit and further comprising a securing mechanism including a lock associated with the joint (14) and a release unit (24) adapted to be selectively activated, wherein the lock retains the joint (14) in a locking position so long as the release unit (24) is not activated,
**characterized in that**
- the drive unit is associated with the joint (14) and is configured to move the joint (14) by driving at least one of the parts (18) of the joint (14) which are movable relative to one another, and **in that**
- the securing mechanism permits a movement of the joint (14) out of the respective locking position within a predetermined, limited range of movement even when the release unit (24) is not activated.

2. Retaining device according to Claim 1,
wherein the retaining device (10) further comprises a mechanism for active vibration damping, which is designed to output to the drive unit a balancing signal causing the drive unit to move the joint (14) in such a manner that vibrations of the retaining device (10) can be balanced out.

3. Retaining device according to Claim 1 or 2,
wherein the lock includes at least one stop surface (26a; 42a, 42b) and at least one counter-stop surface (28a, 28b; 44a, 44b), which can interact in order to retain the joint (14) in the locking position.

4. Retaining device according to one of Claims 1 to 3,
wherein the lock includes a brake (30), in particular an electromagnetic brake, with a movable part (32) which is coupled with the at least one driven part (18) of the joint (14), and wherein the at least one stop surface (42a, 42b) of the lock is arranged on the movable part (32) of the brake (30) and the at least one counter-stop surface (44a, 44b) is arranged on the at least one driven part (18) of the joint (14).

5. Retaining device according to one of Claims 1 to 4,
wherein the lock includes a stop, in particular a stop pin (26), on which the at least one stop surface (26a) is formed and which is adapted to engage with a recess (28) having at least two counter-stop surfaces (28a, 28b) for locking the movement of the joint in two opposite directions of motion (R).

6. Retaining device according to Claim 5,
wherein the stop is movable back and forth between a first position, in which it locks the movement of the joint (14), and a second position, in which it allows the movement of the joint (14).

7. Retaining device according to one of Claims 3 to 6,
wherein a clearance is provided between the stop surface (26a; 42) and the counter-stop surface (28a, 28b; 44a, 44b) of the lock in the direction (R) of motion of the joint (14).

8. Retaining device according to one of Claims 3 to 7,
wherein at least one elastic intermediate element (E) is provided between the stop surface and the counter-stop surface (28a, 28b) of the lock in the direction (R) of motion of the joint (14).

9. Retaining device according to one of Claims 1 to 8,
wherein the retaining device (10) further includes a monitoring device which is configured to monitor whether the joint (14) is being moved within the predetermined range of movement and to transmit a respective feedback to the securing mechanism.

10. Retaining device according to Claim 9,
wherein the securing mechanism can allow an activation of the lock based on the feedback of the monitoring device.

11. Retaining device according to one of Claims 9 or 10,
wherein the monitoring device includes at least one sensor for monitoring the movement of the joint.

12. Method for operating a securing mechanism of a retaining device (10) for an instrument (12), wherein the retaining device (10) includes:
- at least one joint (14), with at least two parts (16, 18) which are movable relative to one another, and at least one drive unit associated with the joint (14), wherein the drive unit is configured to move the joint (14) by driving at least one of the parts (18) of the joint (14) which are movable relative to one another, and
- the securing mechanism including a lock associated with the joint (14) and a release unit (24) adapted to be activated,
wherein the method includes the following steps:
- determining (S100), whether the release unit (24) is activated,
- releasing the lock (S120),
- activating a monitoring means (S130),
- if the release unit (24) is not activated (S110), storing a locking position and monitoring (S140) a movement of the joint (14) as to whether starting from the locking position the joint (14) is moved within a predetermined range of movement,
- if the joint (14) moves beyond the predetermined range of movement, activating the lock (S143).

## Revendications

1. Dispositif de retenue (10) pour un instrument (12), qui présente au moins un joint articulé (14) comportant au moins deux parties (16, 18) mobiles l'une par rapport à l'autre et au moins une unité d'entraînement, et qui présente en outre un moyen de sécurité avec un moyen de blocage associé au joint articulé (14) et avec une unité de libération (24) activable sélectivement, le moyen de blocage maintenant le joint articulé (14) dans une position de blocage tant que l'unité de libération (24) n'est pas activée,
**caractérisé en ce que**
- l'unité d'entraînement est associée au joint articulé (14) et est conçue pour déplacer le joint articulé (14) en entraînant au moins une des parties (18) mobiles l'une par rapport à l'autre du joint articulé (14), et **en ce que**
- le moyen de sécurité autorise un déplacement du joint articulé (14) à l'intérieur d'une zone de déplacement limitée prédéterminée à partir de la position de blocage respective même si l'unité de libération (24) n'est pas activée.

2. Dispositif de retenue selon la revendication 1,
dans lequel le dispositif de retenue (10) présente en outre un moyen d'amortissement actif des vibrations qui est conçu pour délivrer un signal de compensation à l'unité d'entraînement, lequel amène l'unité d'entraînement à déplacer le joint articulé (14) de manière à permettre la compensation des vibrations du dispositif de retenue (10).

3. Dispositif de retenue selon la revendication 1 ou la revendication 2,
dans lequel le moyen de blocage comprend au moins une surface de butée (26a ; 42a, 42b) et au moins une surface de contre-butée (28a, 28b ; 44a, 44b) qui peuvent coopérer pour maintenir le joint articulé (14) dans la position de blocage.

4. Dispositif de retenue selon l'une des revendications 1 à 3,
dans lequel le moyen de blocage comprend un frein (30), en particulier un frein électromagnétique, comportant une partie mobile (32) qui est couplée à ladite au moins une partie entraînée (18) du joint articulé (14), et dans lequel ladite au moins une surface de butée (42a, 42b) du moyen de blocage est disposée sur la partie mobile (32) du frein (30) et ladite au moins une surface de contre-butée (44a, 44b) est disposée sur ladite au moins une partie entraînée (18) du joint articulé (14).

5. Dispositif de retenue selon l'une des revendications 1 à 4,
dans lequel le moyen de blocage présente une butée, en particulier une broche de butée (26), sur laquelle est formée ladite au moins une surface de butée (26a) et qui peut s'engager dans un évidement (28) comportant au moins deux surfaces de contre-butée (28a, 28b) pour bloquer le déplacement du joint articulé dans deux directions de déplacement opposées (R).

6. Dispositif de retenue selon la revendication 5,
dans lequel la butée est mobile alternativement entre une première position dans laquelle elle bloque le déplacement du joint articulé (14) et une seconde position dans laquelle elle autorise le déplacement du joint articulé (14).

7. Dispositif de retenue selon l'une des revendications 3 à 6,
dans lequel un jeu est prévu entre la surface de butée (26a ; 42) et la surface de contre-butée (28a, 28b ; 44a, 44b) du moyen de blocage dans la direction (R) du déplacement du joint articulé (14).

8. Dispositif de retenue selon l'une des revendications 3 à 7,
dans lequel au moins un élément intermédiaire élastique (E) est prévu entre la surface de butée et la surface de contre-butée (28a, 28b) du moyen de blocage dans la direction (R) du déplacement du joint articulé (14).

9. Dispositif de retenue selon l'une des revendications 1 à 8,
dans lequel le dispositif de retenue (10) présente en outre un moyen de surveillance qui est conçu pour surveiller si le joint articulé (14) est déplacé à l'intérieur de la zone de déplacement prédéterminée et pour fournir une information en retour au moyen de sécurité.

10. Dispositif de retenue selon la revendication 9,
dans lequel le moyen de sécurité peut autoriser une activation du moyen de blocage sur la base de l'information en retour du moyen de surveillance.

11. Dispositif de retenue selon l'une des revendications 9 ou 10,
dans lequel le moyen de surveillance présente au moins un capteur pour surveiller le déplacement du joint articulé.

12. Procédé de fonctionnement d'un moyen de sécurité d'un dispositif de retenue (10) pour un instrument (12), le dispositif de retenue (10) comprenant :
- au moins un joint articulé (14) comportant au moins deux parties (16, 18) mobiles l'une par rapport à l'autre et au moins une unité d'entraînement associée au joint articulé (14), l'unité d'entraînement étant conçue pour déplacer le joint articulé (14) en entraînant au moins une des parties (18) mobiles l'une par rapport à l'autre du joint articulé (14), et
- le moyen de sécurité avec un moyen de blocage associé au joint articulé (14) et une unité de libération activable (24),
le procédé comprenant les étapes consistant à :
- vérifier (S100) si l'unité de libération (24) est activée,
- desserrer le blocage (120),
- activer un moyen de surveillance (S130),
- mémoriser, lorsque l'unité de libération (24) n'est pas activée (S110), une position de blocage et surveiller (S140) un déplacement du joint articulé (14) pour voir si le joint articulé (14) est déplacé à l'intérieur d'une zone de déplacement prédéfinie à partir de cette position de blocage,
- activer le moyen de blocage (S143) lorsque le joint articulé (14) se déplace au-delà de la zone de déplacement prédéfinie.
